# EUROPEAN PATENT SPECIFICATION

(11) **EP 1 648 548 B1**
(45) Date of publication and mention of the grant of the patent: **11.06.2008**
(21) Application number: 04778249.5
(22) Date of filing: 14.07.2004
(51) Int. Cl.: A61M 25/00, A61B 17/12

(54) **MEDICAL DEVICES**
MEDIZINISCHE VORRICHTUNGEN
DISPOSITIFS MEDICAUX

(30) Priority: 18.07.2003 US 488644 P
(43) Date of publication of application: 26.04.2006
(73) Proprietor: Boston Scientific Limited, Christ Church (BB)
(72) Inventor: DAO, Kinh-Luan, Randolph, MA 02368 (US); LIU, Changdeng, Storrs, CT 06268 (US); MATHER, Patrick, Chagrin Falls, OH 44022 (US); SAHATJIAN, Ronald, A., Lexington, MA 02420 (US); ZHONG, Sheng-Ping, Northborough, MA 01532 (US)
(74) Representative: Peterreins, Frank
(86) International application number: PCT/US2004/022644
(87) International publication number: WO 2005/009523

(56) References cited:
- US-A- 6 077 256
- US-A- 6 149 664
- US-A- 2002 142 119
- US-B1- 6 358 238

## Description

### TECHNICAL FIELD

The invention relates to medical catheters.

### BACKGROUND

The body includes various blood vessels, for example, arteries. Sometimes, a wall portion of a blood vessel becomes stretched and thin such that the blood vessel develops a bulge, or an aneurysm. An aneurysm is potentially dangerous because it can break open, thereby causing the vessel to bleed. Bleeding can result in a stroke (e.g., in a brain aneurysm) or death.

One method of treating an aneurysm is to fill the aneurysm. For example, the aneurysm can be filled with helically wound coils or braids, sometimes called vaso-occlusive devices. The vaso-occlusive devices can promote formation of a clot and a mass surrounding the devices that fill and seal the aneurysm. As a result, the weakened wall of the vessel is not exposed, e.g., to pulsing blood pressure in the vessel, and the possibility of the aneurysm breaking can be reduced.

The vaso-occlusive devices can be delivered into an aneurysm by endovascular techniques using a guidewire and a catheter. The guidewire is first steered through the body and to the aneurysm. Next, the catheter is slid over the emplaced guidewire and tracked to the aneurysm, e.g., at the mouth of the aneurysm, and the guidewire is removed. The vaso-occlusive devices can then be delivered through a lumen of the catheter and into the aneurysm.

US 2002/142119 A1 discloses a medical catheter according to the precharacterizing portion of claim 1. Optical fibers connected to a controlled light source such as a laser or resistive heating of film ligaments are used to heat the catheter tip comprising a shape memory composite to effect shape change.

US 6 358 238 B1 discloses a catheter having a distal tip portion which is expandable from a first diameter approximating a tapered profile to a second diameter approximating a nominal diameter of the distal shaft portion. The expansion of the distal tip portion is accomplished by the application of pressure. In addition to pressure, the distal tip portion may be expanded using shape memory polymers.

US 6 149 664 A discloses an introducer for retaining an implant placed within an implant delivery device having a pusher for displacing the implant which includes a tube and a clamp. The interior of the tube defines a passage for guiding the movement of the pusher. The clamp is made of a shape memory material disposed in the passage about the pusher. The clamp is normally operative to grip the pusher to hold it in position relative to the tube and further operative in response to an elevated temperature to release the pusher for movement relative to the tube. The clamp may be formed integrally with the tube and may comprise at least one crimp or annular ring surrounding the tube. The tube is tapered to be narrower at its distal end and may be formed of a shape memory polymer material

### SUMMARY

The invention relates to a medical catheter as defined in claim 1.

Advantageous embodiments of the invention are defined in dependent claims 2 to 12.

In one aspect, the invention features a catheter having a portion, e.g., a distal portion, including a shape memory polymer. The catheter can be delivered to a target site, e.g., near an aneurysm, in a first configuration; and at the target site, the portion of the catheter can be changed to a second, different configuration. For example, the catheter can be delivered with the distal portion in a straight position, and subsequently, the distal portion can be changed to a bent configuration that facilitates delivery of vaso-occlusive devices into the aneurysm.

The shape memory polymer portion allows the catheter to be delivered, for example, without relying on a guidewire to straighten the catheter and/or without being deformed by a tortuous vasculature. After changing configuration, the shape memory polymer portion provides a stable (e.g., non-slipping) pathway for delivery of the vaso-occlusive devices. By securely staying in the predetermined changed configuration, the catheter reduces the likelihood of buckling or other forces that can exert stress on the aneurysm.

In another aspect, the invention features a medical catheter including a tubular member having a first portion including a shape memory polymer.

Embodiments can include one or more of the following features. The first portion is a distal portion of the tubular member, or a distalmost portion of the tubular member. The tubular member has a body including a polymer different than the shape memory polymer. An end of the body can be connected to an end of the first portion. The first portion surrounds a portion of the body.

The shape memory polymer can include, for example, polynorbonene, polycaprolactone, polyene, nylon, polycyclooctene (PCO), a blend of polcyclooctene and styrenebutadiene rubber, polyurethane, polyurethane copolymers, and/or a polyvinyl acetate/polyvinylidinefluoride.

The catheter can be in the form of a 5 French catheter or smaller.

The first portion can include a radiopaque material, a material visible by magnetic resonance imaging, and/or an ultrasound contrast agent.

Other aspect, features, and advantages of the invention will be apparent from the description of the preferred embodiments thereof and from the claims.

### DESCRIPTION OF DRAWINGS

Fig. 1 is an illustration of an embodiment of a method treating an aneurysm.
Fig. 2A is an illustration of an embodiment of a catheter in a generally straight position; and Fig. 2B is an illustration of the catheter of Fig. 2A in a bent position.
Fig. 3A is an illustration of an embodiment of a catheter in a generally straight position; and Fig. 3B is an illustration of the catheter of Fig. 3A in a bent position.

### DETAILED DESCRIPTION

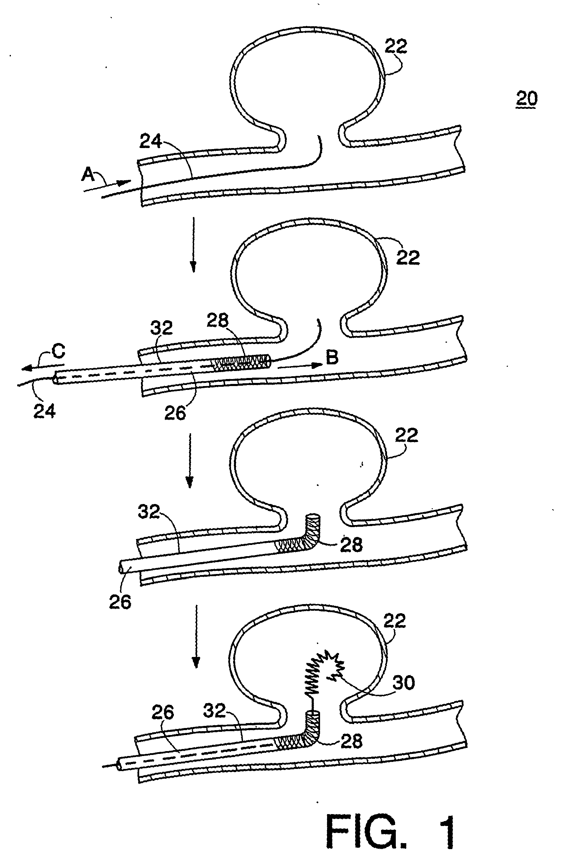

Referring to Fig. 1, a method 20 of treating an aneurysm 22 is shown. Method 20 includes delivering a guidewire 24 (e.g., a steerable guidewire) to aneurysm 22 using conventional endovascular techniques (arrow A). Next, a catheter 26 is passed over guidewire 24, and advanced to near aneurysm 22 (arrow B). Catheter 26 includes a body 32 and a distal portion 28 including a shape memory polymer that is configured to remember a predetermined configuration. During advancement of catheter 26, distal portion 28 is in a generally straight configuration (e.g., collinear with body 32), which allows the catheter to easily track a tortuous vascular path. Guidewire 24 is then removed (e.g., withdrawn proximally, arrow C). Next, distal portion 28 is changed from the straight configuration to the predetermined configuration (as shown, a bent configuration). Distal portion 28 is then introduced into aneurysm 22, and vaso-occlusive coils 30 are introduced through catheter 26 and into the aneurysm, according to conventional methods.

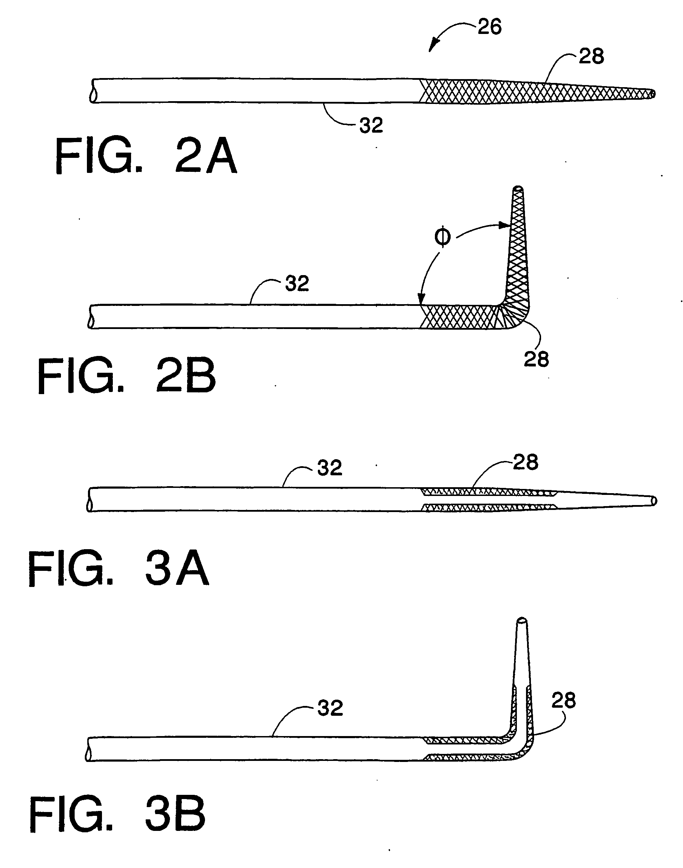

Catheter 26 is generally an elongated tube having one or more lumens. Referring to Figs. 2A and 2B, catheter 26 generally includes body 32 and distal portion 28. Body 32 can be a standard catheter shaft made of conventional, biocompatible polymers, as described in, e.g., U.S.S.N. 09/798,749, filed March 2, 2001, and entitled "Multilayer Medical Balloon", published as US 2002/0165523 on November 7, 2002. As shown, distal portion 28 is an extruded tube having a tapered portion. In some embodiments, referring to Figs. 3A and 3B, distal portion 28 is a sleeve that fits over a distal portion of body 32. Body 32 can have a tapered distal end. Distal portion 28 can be attached to body 32, for example, by laser welding, gluing with an epoxy, melt bonding, or heat shrinking. In other embodiments, distal portion 28 can be a coating of a shape memory polymer applied to body 32, e.g., by dipping the body into a solution containing a shape memory polymer. Distal portion 28 can be, for example, about six to ten inches long.

Distal portion 28 includes one or more shape memory polymers (SMPs). Suitable shape memory polymers include elastomers that exhibit melt or glass transitions at temperatures that are above body temperature, e.g., at about 40 to 50 °C, and safe for use in the body. Examples of polymers include shape memory polyurethanes (available from Mitsubishi), polynorbomene (e.g., Norsorex (Mitsubishi)), polymethylmethacrylate (PMMA), poly(vinyl chloride), polyethylene (e.g., crystalline polyethylene), polyisoprene (e.g., trans-polyisoprene), styrene-butadiene copolymer, rubbers, or photocrosslinkable polymer including azo-dye, zwitterionic and other photochromic materials (as described in Shape Memory Materials, Otsuka and Wayman, Cambridge University Press, 1998). Other shape memory polymers include shape memory plastics available from MnemoScience GmbH Pauwelsstrasse 19, D-52074 Aachen, Germany. The materials can be bioabsorbable or non-bioabsorbable. Mixtures of polymeric shape memory materials can be used.

In some embodiments, the shape memory polymer is crosslinked and/or crystalline. The degree of crosslinking and/or crystallinity is sufficient to resist excessive creep or stress relaxation, e.g., after the polymer is heated. Crosslinking can also be controlled to adjust the melt or glass transition temperature and transition temperature range. In some cases; a narrow transition range, e.g. 10°C, 5 °C, or less, is desirable. Crosslinking can be achieved by application of radiation, such as e-beam, UV, gamma, x-ray radiation, or by heat-activated chemical crosslinking techniques (e.g., with peroxides). In some radiation-crosslinking techniques, the polymer need not be substantially heated to achieve crosslinking

As noted above, the shape memory polymer is capable of exhibiting shape memory properties such that it can be configured to remember, e.g., to change to, a predetermined configuration or shape. In some embodiments, the shape memory polymer is formed or set to a primary (e.g., stress free) shape during crosslinking. For example, distal portion 28 can be crosslinked in a bent configuration. Subsequently, the polymer can be formed into a temporary shape, for example, by heating the polymer to a softening point (e.g., Tₘ or T_{g}), deforming the polymer, and cooling the polymer to below a softening point. When the polymer is subsequently heated to above the softening temperature, the polymer can recover to its primary form.

The polymer is compounded to include a material, such as magnetic particles, that is susceptible to heating by magnetic effects, such as hysteresis effects. A magnetic field can be imposed on the stent body by a source on a catheter or outside the body. Suitable magnetic particles are available as the Smartbond^{™} System from Triton Systems, Inc., Chelmsford, MA. Heating by magnetic effects is discussed in U.S. Patent No. 6,056,844.

In general, the size and configuration of catheter 26 is not limited. In some embodiments, catheter 26 is in the form of a 5 French catheter or smaller, e.g., a 4 French, 3 French, 2 French, or 1 French catheter. Catheter 26 can have a length of, for example, about 240 cm to about 3.5 meters. Examples of catheters include aneurysm catheters, guide catheters, urology catheters, and microcatheters (all available from Boston Scientific Corp., Natick, MA).

The angle at which distal portion 28 can be bent relative to body 32 can also vary. In some cases, the angle (φ) defined by distal portion 28 and body 32 (Fig. 2B) is between about 20° and about 180°. For example, the angle (φ) can be greater than or equal about 20°, 40°, 60°, 80°, 100°, 120°, 140°, or 160°; and/or less than or equal to about 180°, 160°, 140°, 120°, 100°, 80°, 60°, or 40°.

In some embodiments, distal portion 28 contains a radiopaque material, a material that is visible by magnetic resonance imaging (MRI), and/or an ultrasound contrast agent. The materials or agent allows catheter 26 to be tracked and monitored, e.g., by X-ray fluoroscopy, MRI, or ultrasound imaging. Examples of radiopaque materials include tantalum, tungsten, platinum, palladium, or gold. The radiopaque material, e.g., powder, can be mixed with the shape memory polymer. Alternatively or in addition, the radiopaque material, e.g., a band of radiopaque material, can be placed on catheter 26 at selected positions, such as, for example, adjacent to distal portion 28.

Examples of MRI visible materials include non-ferrous metal-alloys containing paramagnetic elements (e.g., dysprosium or gadolinium) such as terbium-dysprosium, dysprosium, and gadolinium; non-ferrous metallic bands coated with an oxide or a carbide layer of dysprosium or gadolinium (e.g., Dy₂O₃ or Gd₂O₃); non-ferrous metals (e.g., copper, silver, platinum, or gold) coated with a layer of superparamagnetic material, such as nanocrystalline Fe₃O₄, CoFe₂O₄, MnFe₂O₄, or MgFe₂O₄; and nanocrystalline particles of the transition metal oxides (e.g., oxides of Fe, Co, Ni). Powder of MRI visible materials can be mixed with the shape memory polymer.

The ultrasound contrast agent can be any material that enhances visibility during ultrasound imaging. An ultrasound contrast agent can include a suspension having trapped bubbles of sufficient size to deflect sound waves.

Distal portion 28 can include a drug or a therapeutic agent. For example, distal portion 28 can include an antithrombolytic agent, such as heparin, to reduce clotting on the catheter. Other examples of drugs or therapeutic agents are described in U.S.S.N. 10/232,265, filed August 30, 2002 published as US 2003/0185895 on October 3, 2003.

In some cases, the catheter can be formed into a desired shape by a user (such as a physician) at the time of a procedure, e.g., using heat (steam). The shape memory properties can be used to impart a predetermined shape, which the user can try. If the predetermined shape is not adequate (e.g., unsuccessful), the user can heat the shape memory material of the catheter outside the body and re-shape the catheter (e.g., using steam or hot water) to a second predetermined shape.

The following examples are illustrative and not intended to be limiting.

### Example 1

The following example shows a method of making a catheter having portion including a polycyclooctene/styrene butadiene rubber blend, a blend of shape memory polymers. Polycyclooctene and blends of shape memory polymers are described in U.S.S.N. 10/683,559, entitled "Crosslinked Polycyclooctene" published as US 2004/0122184 on June 24, 2004, and U.S.S.N. 10/683,558, entitled "Blends of Amorphous and Semicrystalline Polymers Having Shape Memory Properties" published as US 2004/0122174 on June 24, 2004. both filed on October10, 2003.

To form the blend, the polymers were compounded. Styrene butadiene rubber was cut in a Willy mill to 1-2 mm mesh. The rubber and the polycyclooctene were mixed in a ratio of 65% by weight polycyclooctene and 35% by weight styrene butadiene rubber, and ran in a dry blender for about 30 minutes. The mixture was then placed in a bra blender with two twin-screw head running at 20-25 RPM at 100°C. The blended mixture was then placed in a room temperature water bath and pelletized.

Next, the pellets were extruded. The pellets (about 500 grams) were extruded in a Davis Standard extruder (3/4 to one inch) running with a feed temperature of about 50 °C, a second zone at about 65 °C, a third zone at about 80 °C, and a die head temperature of about 80 °C. The pellets were extruded through a tubular die and using pressurized air to help maintain the patency of the lumen of the extruded tube. The extruded tube was fed to a room temperature water bath and cut to length (e.g., about 15,24 cm (6 inches)). The tube can have, for example, a 0,0762 cm (0.0305 inch) O.D. and a 0,0686 cm (0.027 inch) I.D.

Next, the shape memory polymer tube (e.g., about 5 cm) was placed on a distal portion of a catheter. The catheter had a guidewire placed through the lumen of the catheter. The catheter was attached to a sleeving machine equipped with movable and heatable clamps. Next, a heat shrink tubing (available from Zeus or Target) was placed over the shape memory polymer tube. The clamps were heated to about 175°C to about 200°C and moved at a rate of 13.5-17 cm/min to shrink the heat shrink tubing and to secure the shape memory tubing to the catheter. Typically, only one pass of the clamps is needed. In some cases, the higher the concentration of styrene butadiene rubber in the blend, the higher the temperature is needed; for example, a blend including 35% by weight styrene butadiene was heated to about 195 °C.

Then, the catheter was removed from the sleeving machine, and the heat shrink tubing was stripped from the catheter under a microscope.

The catheter was then shaped, e.g., into a curve (e.g., J shape) or a straight line. A shaping mandrel can be placed in the lumen of the catheter.

The catheter was then sent to a facility (such as Steris Isomedix) for crosslinking with gamma radiation. Depending on the thickness of the shape memory polymer portion, the polymer portion was irradiated with between about 1 and about 25 megarads. For microcatheters, about 1 megarad was irradiated.

### Example 2

A shape memory polymer solution was prepared by dissolving fifteen grams of polynorbonene (Nosorex, from Mitsubishi) and three grams of Kraton 1650 G (GLS Corp.) in 500 mL of xylene. The solution was heated to about 70 °C and stirred on a magnetic stirring plate at a low setting (about 50 rpm) for about 30 minutes.

A curved mandrel was inserted into a catheter (Imager catheter (urology) or Renegade catheter (neurology), available from Boston Scientific Corp.) to provide a curved catheter. The curved catheter, about 15,24-25,4 cm (about 6-10 inches) was dipped into the shape memory solution. Depending on the rate at which the catheter was withdrawn from the solution, it is believed that the thickness of the shape memory polymer is between about 0,0025-0,0127 cm (0.001-0.005) inch thick. The catheter was air dried for about twenty minutes. The mandrel was then removed.

The curved catheter was straightened by immersing the catheter in a 50°C water bath.

The straight catheter can be returned to its curved shape by heating the catheter above body temperature, e.g., 45-50 °C.

Other embodiments are within the claims.

## Claims

1. A medical catheter comprising:
a tubular member comprising a body (32) and a first portion (28) comprising a shape memory polymer the first portion (28) being adapted to change to a bent configuration, **characterized in that** the first portion (28) further comprises a material susceptible to heating by magnetic effects to heat said shape memory polymer to change the first portion (28) into said bent configuration.

2. The catheter of claim 1, wherein the first portion is a distal portion of the tubular member.

3. The catheter of claim 1, wherein the first portion is a distalmost portion (28) of the tubular member.

4. The catheter of claim 1, wherein the shape memory polymer comprises a material selected from the group consisting of a polynorbonene, a polycaprolactone, a polycyclooctene, a polycyclooctene/styrene butadiene blend, and a polyvinyl acetate/polyvinylidinefluoride.

5. The catheter of claim 1, wherein the said material (28) comprises magnetic particles susceptible to heating by hysteresis effects.

6. The catheter of claim 1, wherein the body (32) comprises a polymer different than the shape memory polymer.

7. The catheter of claim 6, wherein an end of the body (32) is connected to an end of the first portion (28).

8. The catheter of claim 6, wherein the first portion (28) surrounds a portion of the body (32).

9. The catheter of claim 1, in the form of a 5 French catheter or smaller.

10. The catheter of claim 1, wherein the first portion (28) further comprises a radiopaque material.

11. The catheter of claim 1, wherein the first portion (28) further comprises a material visible by magnetic resonance imaging.

12. The catheter of claim 1, wherein the first portion (28) further comprises an ultrasound contrast agent.

## Patentansprüche

1. Medizinischer Katheter, umfassend:
ein röhrenförmiges Element, das einen Körper (32) umfasst und einen ersten Teilbereich (28), der ein Formgedächtnispolymer umfasst,
wobei der erste Teilbereich (28) dafür geeignet ist, in einen gebogenen Zustand versetzt zu werden;
**dadurch gekennzeichnet, dass** der erste Teilbereich (28) des weiteren ein Material umfasst, das mit Hilfe von Magnetwirkung zu erwärmen ist, um das Formgedächtnispolymer zu erwärmen, so dass der erste Teilbereich (28) in den gebogenen Zustand versetzt werden kann.

2. Katheter gemäß Anspruch 1, wobei der erste Teilbereich ein Endbereich des röhrenförmigen Elements ist.

3. Katheter gemäß Anspruch 1, wobei der erste Teilbereich der äußerste Endbereich (28) des röhrenförmigen Elements ist.

4. Katheter gemäß Anspruch 1, wobei das Formgedächtnispolymer ein Material umfasst, das aus einer Werkstoffgruppe bestehend aus einem Polynorbonen, einem Polycaprolacton, einem Polycycloocten, einem Polycycloocten/Styrol-Butadien-Gemisch und einem Polyvinylacetat/Polyvinylidenfluorid ausgewählt wird.

5. Katheter gemäß Anspruch 1, wobei das Material (28) Magnetpartikel umfasst, die mit Hilfe von Hystereseeffekten leicht zu erwärmen sind.

6. Katheter gemäß Anspruch 1, wobei der Körper (32) ein Polymer umfasst, das sich vom Formgedächtnispolymer unterscheidet.

7. Katheter gemäß Anspruch 6, wobei ein Ende des Körpers (32) mit einem Ende des ersten Teilbereichs (28) verbunden ist.

8. Katheter gemäß Anspruch 6, wobei der erste Teilbereich (28) einen Teil des Körpers umgibt.

9. Katheter gemäß Anspruch 1 mit einem Außendurchmesser von maximal 5 French.

10. Katheter gemäß Anspruch 1, wobei der erste Teilbereich (28) des weiteren ein röntgenkontrastgebendes Mittel umfasst.

11. Katheter gemäß Anspruch 1, wobei der erste Teilbereich (28) des weiteren ein Mittel umfasst, das bei einer Magnetresonanztomographie sichtbar ist.

12. Katheter gemäß Anspruch 1, wobei der erste Teilbereich (28) des weiteren ein Ultraschallkontrastmittel umfasst.

## Revendications

1. Cathéter médical comprenant :
un élément tubulaire comprenant un corps (32) et une première portion (28) comprenant un polymère à mémoire de forme,
la première portion (28) étant apte à se modifier pour prendre une configuration cintrée,
**caractérisé en ce que** la première portion (28) comprend, de plus, un matériau sensible au chauffage par des effets magnétiques pour chauffer ledit polymère à mémoire de forme et modifier la première portion (28) en ladite configuration cintrée.

2. Cathéter selon la revendication 1, dans lequel la première portion est une portion distale de l'élément tubulaire.

3. Cathéter selon la revendication 1, dans lequel la première portion est une portion distale extrême (28) de l'élément tubulaire.

4. Cathéter selon la revendication 1, dans lequel le polymère à mémoire de forme comprend un matériau choisi dans le groupe consistant en un polynorbonène, une polycaprolactone, un polycyclooctène, un mélange polycyclooctène/styrène butadiène et un acétate de polyvinyle/fluorure de polyvinylidine.

5. Cathéter selon la revendication 1, dans lequel ledit matériau (28) comprend des particules magnétiques sensibles au chauffage par des effets d'hystérésis (32).

6. Cathéter selon la revendication 1, dans lequel le corps (32) comprend un polymère différent du polymère à mémoire de forme.

7. Cathéter selon la revendication 6, dans lequel une extrémité du corps (32) est raccordée à une extrémité de la première portion (28).

8. Cathéter selon la revendication 6, dans lequel la première portion (28) entoure une portion du corps (32).

9. Cathéter selon la revendication 1, sous la forme d'un cathéter français 5 ou plus petit.

10. Cathéter selon la revendication 1, dans lequel la première portion (28) comprend, de plus, un matériau radio-opaque.

11. Cathéter selon la revendication 1, dans lequel la première portion (28) comprend, de plus, un matériau visible par imagerie à résonance magnétique.

12. Cathéter selon la revendication 1, dans lequel la première portion (28) comprend, de plus, un agent de contraste aux ultrasons.
